# EUROPEAN PATENT APPLICATION

(11) **EP 4 650 444 A1**
(43) Date of publication of application: **19.11.2025**
(21) Application number: 23916422.1
(22) Date of filing: 12.12.2023
(51) Int. Cl.: C12N 15/10, B01L 3/00, C12Q 1/6806, C12Q 1/6844

(54) **NUCLEIC ACID EXTRACTION VESSEL PROVIDED WITH POSITIVELY CHARGED POLYMER THIN FILM, AND SINGLE-POT NUCLEIC ACID DETECTION METHOD USING CHARGE TRANSFER POLYPLEX**

(30) Priority: 13.01.2023 KR 20230005511
(71) Applicant: Korea Advanced Institute of Science and Technology, Daejeon 34141 (KR)
(72) Inventor: LIM, Sung Gap, Daejeon 34141 (KR); KIM, Seongeun, Daejeon 34141 (KR); PARK, Nahyun, Daejeon 34141 (KR); JEONG, Booseok, Daejeon 34141 (KR); SONG, Younseong, Daejeon 34141 (KR); LEE, Kyoung Gun, Daejeon 34140 (KR); PARK, Yu Min, Cheongju-si Chungcheongbuk-do 28330 (KR); JO, Da Ae, Gumi-si Gyeongsangbuk-do 39179 (KR); KANG, Tae Joon, Daejeon 34140 (KR); SONG, Ja Yeon, Daejeon 34140 (KR); JANG, Hyo Won, Daejeon 35233 (KR)
(74) Representative: Viering, Jentschura & Partner mbB Patent- und Rechtsanwälte
(86) International application number: PCT/KR2023/020425
(87) International publication number: WO 2024/150939

(57) **Abstract**

A nucleic acid detection method comprises the steps of: inputting a sample containing nucleic acids into a vessel coated with a thin film comprising a cationic polymer having a hydrolyzable side chain; forming a polymer-nucleic acid complex by binding the nucleic acids to the cationic polymer which has been dissolved from the thin film; releasing the nucleic acids from the polymer-nucleic acid complex by hydrolyzing the cationic polymer; and amplifying the nucleic acids or other nucleic acids synthesized from said nucleic acids. The nucleic acid detection method may be carried out in a single pot, may reduce the detection time, and may improve detection accuracy.

## Description

### TECHNICAL FIELD

The present invention relates to nucleic acid extraction. More particularly, the present invention relates to a nucleic acid extraction vessel provided with a positively charged polymer thin film and a single-pot nucleic acid detection method using a charge transfer polyplex.

### BACKGROUND

Recently, spread of highly contagious and toxic infectious diseases has threatened global health and the economy. Accordingly, demand for technology to quickly and accurately diagnose diseases has increased to prevent spread of infectious diseases and reduce damage. In the case of diseases caused by viruses, rapid diagnosis is even more essential because many variants occur due to characteristics of viruses that frequently mutate. Currently, reverse transcription quantitative PCR (RT-qPCR) is frequently used as a diagnostic method, and it can amplify various pathogen genes as well as corona virus, enabling the diagnosis of various diseases.

A molecular diagnostic process consists of sample collection, gene extraction, gene amplification, and detection processes. In particular, a gene extraction process is complicated (it includes virus lysis, virus gene capture with solid matrix, washing, and gene release), which consumes a lot of time and reagents. An important step in the gene extraction process is gene capture and release (elution). In order to accurately and quickly diagnose, it is necessary to capture and release genes with high efficiency before the amplification step. Currently known gene capture methods using silica, magnetic beads, etc. can achieve relatively high capture efficiency, but the elution efficiency is low. In order to increase the efficiency of gene extraction, strategies such as using chaotropic reagents or increasing pH or temperature have been used, but these methods may have a negative effect on the efficiency of the subsequent amplification step and have low compatibility.

### [Prior Arts]

### [Patent literature]

### 1. Korean Patent Publication No. 10-2020-0092691

### [Non-patent literature]

1. Song, Younseong, et al. "All-in-One DNA Extraction Tube for Facilitated Real-Time Detection of Infectious Pathogens." Advanced Healthcare Materials 10.14 (2021): 2100430.

### DETAILED DESCRIPTION OF THE INVENTION

### TECHNICAL PROBLEM TO BE SOLVED

One object of the present invention is to provide a polymer thin film capable of capturing and releasing nucleic acids (DNA or RNA) with high efficiency.

Another object of the present invention is to provide a method for extracting nucleic acids using the polymer thin film.

However, the object to be solved by the present invention is not limited to the object mentioned above, and may be expanded in various ways without departing from the spirit and scope of the present invention.

### MEANS FOR SOLVING PROBLEM

A nucleic acid extraction vessel according to an embodiment of the present invention includes a body for containing a sample, and a polymer thin film coated on an inner surface of the body. The polymer thin film includes a cationic polymer having a hydrolyzable side chain, and has a surface potential of 1 mV to 50 mV, and a hydrolysis efficiency of the cationic polymer is 30% or more upon when reacted at 50 °C for 1 hour.

According to an embodiment, the hydrolyzable side chain includes a hydrolytic cationic ester.

According to an embodiment, the cationic polymer includes a repeating unit polymerized from a monomer including at least one of 2-(dimethylamino)ethyl acrylate (DMAEA), 2-(dimethylamino)ethyl methacrylate (DMAEMA), and 2-(diethylamino)ethyl methacrylate (DEAEMA).

According to an embodiment, the cationic polymer includes a repeating unit polymerized from DMAEA.

According to an embodiment, the cationic polymer is a linear polymer.

According to an embodiment, a number average molecular weight of the cationic polymer is 1,000 g/mol to 3,000 g/mol.

A nucleic acid extraction vessel according to an embodiment of the present invention includes a body having a containing space, and a polymer thin film coated on an inner surface of the body to contact the containing space. The polymer thin film includes a cationic polymer having a hydrolyzable side chain, which includes a hydrolytic cationic ester, and having a number average molecular weight of the cationic polymer is 1,000 g/mol to 3,000 g/mol.

A nucleic acid detection method according to an embodiment of the present invention includes inputting a sample containing a nucleic acid into a vessel coated with a thin film including a cationic polymer having a hydrolyzable side chain, combining the cationic polymer dissolved from the thin film with the nucleic acid to form a polymer-nucleic acid complex, hydrolyzing the cationic polymer to release the nucleic acid from the polymer-nucleic acid complex, and amplifying the nucleic acid or another nucleic acid synthesized from the nucleic acid.

According to an embodiment, hydrolyzing the cationic polymer to release the nucleic acid is performed at 30 °C to 60 °C.

According to an embodiment, the nucleic acid includes an RNA.

According to an embodiment, the method further includes synthesizing a complementary DNA (cDNA) of the RNA through a reverse transcription reaction, and amplifying the nucleic acid or another nucleic acid synthesized from the nucleic acid amplifies the cDNA.

According to an embodiment, the nucleic acid is released when the cDNA is synthesized.

According to an embodiment, the cationic polymer includes a repeating unit polymerized from DMAEA, and a number average molecular weight of the cationic polymer is 1,000 g/mol to 3,000 g/mol.

According to an embodiment, forming the polymer-nucleic acid complex, releasing the nucleic acid, and amplifying the nucleic acid or another nucleic acid synthesized from the nucleic acid are performed in the same vessel.

### EFFECTS OF THE INVENTION

According to the embodiments of the present invention, a nucleic acid extraction vessel includes a thin film of a cationic polymer. The cationic polymer dissolved from the thin film three-dimensionally captures a nucleic acid, thereby expanding an area for polymer-nucleic acid interaction. Therefore, the capture speed and the capture efficiency for the nucleic acid may be improved.

In addition, the nucleic acid may be released by electrostatic repulsion between the polymer and the nucleic acid by self-catalytic hydrolysis of the polymer. Therefore, more nucleic acids may be released at a lower temperature than the conventional nucleic acid release method without changing conditions that may affect the amplification step (for example, pH 10, 95°C or adding chaotropic reagent).

In addition, the polymer has stability in an amplification step following the nucleic acid extraction step.

Therefore, a single-pot detection system that performs the nucleic acid extraction step and the amplification step continuously may be implemented.

In addition, a nucleic acid detection method using the vessel may shorten detection time of a nucleic acid detection method such as qRT-PCR by capturing nucleic acids in a short time.

In addition, the nucleic acid detection method using the vessel may improve accuracy of a test by high nucleic acid extraction efficiency.

### BRIEF DESCRIPTION OF THE DRAWINGS

FIG. 1 is a side view of a nucleic acid extraction vessel including a polymer thin film according to an embodiment of the present invention.
FIGS. 2a, 2b, 2c, and 2d are schematic diagrams for explaining a nucleic acid extraction method using a nucleic acid extraction vessel according to an embodiment of the present invention.
FIG. 3a is a flowchart for explaining a nucleic acid detection method according to an embodiment of the present invention. FIG. 3b is a schematic diagram for explaining a nucleic acid detection method according to an embodiment of the present invention.
FIG. 4 is a graph showing the results of FTIR (Fourier transform infrared) analysis of the DMAEA polymer (pDMAEA) and monomer (DMAEA) of Synthesis Example 1.
FIG. 5 is a graph showing the results of GPC (Gell permeation chromatography) analysis of the DMAEA polymer of Synthesis Example 1.
FIG. 6 is a graph showing the hydrolysis efficiency (%) over time (at 5-minutes intervals) when the DMAEA polymer of Synthesis Example 1 was hydrolyzed at pH 8.0, 25°C, and 50°C.
FIG. 7 is a graph showing the zeta-potential before and after hydrolysis of the DMAEA polymer of Synthesis Example 1.
FIG. 8 is a graph showing the zeta-potential according to the ratio of the DMAEA polymer and RNA (N(nitrogen)/P(phosphate) ratio) of Synthesis Example 1.
FIG. 9 is a graph showing the average hydrodynamic diameter (Z-average) according to the ratio of the DMAEA polymer and RNA (N(nitrogen)/P(phosphate) ratio) of Synthesis Example 1.
FIG. 10 is a graph showing the capture efficiency, capture amount, recovery rate, and extraction rate in the nucleic acid capture experiment using the tube coated with the DMAEA polymer of Synthesis Example 1.
FIG. 11 shows the detection results of H1N1 using the detection method (PAD-qRT-PCR) of the present invention.
FIG. 12 shows the results of analyzing clinical samples (influenza virus) using the detection method (PAD-qRT-PCR) of the present invention.
FIG. 13 shows a schematic diagram of SARS-CoV-2 and the results of the detection experiment.
FIG. 14 shows the results of analyzing clinical samples (SARS-CoV-2) using the detection method of the present invention (PAD-qRT-PCR) and the conventional detection method (qRT-PCR).
FIG. 15 shows the results of analyzing clinical samples (SARS-CoV-2 variants) using the detection method (PAD-qRT-PCR) of the present invention.

### BEST EMBODIMENT FOR IMPLEMENTING THE INVENTION

Hereinafter, a nucleic acid extraction vessel and a single-pot nucleic acid extraction method using the same according to embodiments of the present invention will be described in detail with reference to the attached drawings. The present invention can be modified in various ways and can take various forms, and specific embodiments will be illustrated and described in detail in the text. However, this is not intended to limit the present invention to a specific disclosed form, and it should be understood that it includes all modifications, equivalents, or substitutes included in ideas and technical scope of the present invention. In the attached drawings, the dimensions of structures are illustrated in an enlarged manner compared to the actual size for the clarity of the present invention.

The terms used in this application are used only to describe specific embodiments and are not intended to limit the present invention. The singular expression includes the plural expression unless the context clearly indicates otherwise. In this application, the terms "include" or "have" are intended to indicate the presence of a feature, number, step, operation, component, or combination thereof described in the specification, but should be understood as not excluding in advance the possibility of the presence or addition of one or more other features, numbers, steps, operations, components, or combinations thereof.

Unless otherwise defined, all terms used herein, including technical or scientific terms, have the same meaning as generally understood by a person of ordinary skill in the art to which the present invention pertains. Terms defined in commonly used dictionaries should be interpreted as having a meaning consistent with the meaning they have in the context of the relevant technology, and shall not be interpreted in an ideal or excessively formal meaning unless explicitly defined in this application.

### Nucleic acid extraction vessel and nucleic acid extraction method

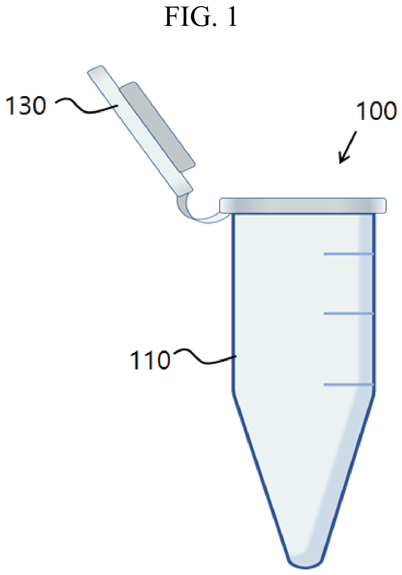

FIG. 1 is a side view of a nucleic acid extraction vessel including a polymer thin film according to an embodiment of the present invention.

Referring to FIG. 1, the nucleic acid extraction vessel 100 may have a tube shape with one open end. For example, the nucleic acid extraction vessel 100 may include a body 110, which has a tube shape and is configured to accommodate a sample, and a cap 130. The cap 130 may close an opening of a top end to seal the vessel.

The nucleic acid extraction vessel according to embodiments of the present invention is not limited to a tube having a cap. For example, the nucleic acid extraction vessel according to embodiments of the present invention may have a tube shape without a cap, or may have various shapes that can accommodate a sample, such as a well plate.

According to an embodiment, a polymer thin film is coated on a surface of the body 110. Therefore, the body 110 may serve as a substrate that supports the polymer thin film. For example, the polymer thin film may be coated on an inner surface of the body 110. According to an embodiment, the polymer thin film may cover the entire inner surface of the body 110. However, embodiments of the present invention are not limited thereto, and the polymer thin film may be disposed on a portion of the inner surface of the body 110, for example, on the bottom surface.

For example, the body 110 may include polypropylene (PP), polyethylene (PE), polyvinyl chloride (PVC), polycarbonate (PC), polyester, polymethylmethacrylate (PMMA), polyethylene terephthalate (PET), polyurethane (PU), silicon, glass, quartz, ceramic, Teflon, or a combination thereof.

The polymer thin film includes a polymer. The polymer may be defined as including an oligomer and/or a polymer having a molecular weight greater than that of an oligomer. The polymer may be a homopolymer having the same repeating unit, or a copolymer having two or more different repeating units.

The polymer thin film includes a cationic polymer, and has a positive surface potential. For example, a surface potential of the polymer thin film may be, but is not limited to, 1 mV to 50 mV, and preferably may be 10 mV or more, for example, 10 mV to 40 mV.

For example, the polymer may include a repeating unit having a positively charged nitrogen in a side chain. In an embodiment, the polymer may include at least a repeating unit having a tertiary amine at an end of the side chain.

The polymer thin film includes a hydrolyzable polymer. For example, the polymer may have a hydrolyzable side chain. The hydrolysis efficiency (%) of the polymer may be defined as the ratio of hydrolyzed repeating units to the repeating units of the entire polymer, and may be 30% or more when reacted at 50 °C for 1 hour. The higher the hydrolysis efficiency, the higher the nucleic acid release efficiency of the polymer, and the shorter the hydrolysis time, the less time required for nucleic acid extraction. Preferably, the hydrolysis efficiency of the polymer may be 30% to 100% when reacted at 50 °C for 30 minutes, and more preferably, 30% or more when reacted at 50 °C for 5 minutes. For example, the hydrolysis efficiency may be measured at about pH 8. The pH conditions are intended to provide a standard for measuring the hydrolysis efficiency, and the performance conditions of the present invention are not limited thereby, and the polymer of the present invention is hydrolyzable in various pH ranges.

The hydrolysis efficiency of the polymer may vary depending on the structure of the polymer, the molecular weight of the polymer, the chemical properties of the side chain, steric hindrance due to the side chain, etc.

According to an embodiment, the polymer may be a linear polymer. Since a linear polymer has high solubility and many hydrolyzable side chains, it can increase the capture-release efficiency of nucleic acids. According to another embodiment, the polymer may have a branched structure or a cross-linked structure. When the polymer has a branched structure or a cross-linked structure, the molecular weight increases and the precipitation speed in a centrifugation process increases, which may reduce the overall nucleic acid extraction time.

For example, the number average molecular weight of the polymer may be 1,000 to 3,000. When the molecular weight of the polymer is excessively small, the size of the complex (polyplex) formed by binding the polymer to the nucleic acid may be too small, thereby making separation and purification difficult. In addition, when the molecular weight of the polymer is excessively large, the solubility increases and the hydrolysis efficiency decreases, which can reduce the capture-release efficiency of nucleic acids. When the polymer has a branched structure or a cross-linked structure, it may have a larger molecular weight than the linear polymer. For example, the number average molecular weight of the linear polymer may be 1,000 to 2,000, and the number average molecular weight of the polymer having a branched structure or a cross-linked structure may be 1,500 to 3,000. However, the embodiments of the present invention are not limited thereto, and may have a molecular weight in a variety of ranges within a range capable of achieving an appropriate hydrolysis efficiency.

According to an embodiment, the polymer may include a hydrolytic cationic ester group as a side chain. The hydrolytic cationic ester group is capable of self-catalyzed hydrolysis by intramolecular interaction between the cationic group (e.g., a dialkylamino group) and the ester carbonyl group.

For example, the polymer may include poly[2-(dimethylamino)ethyl acrylate] (p-DMAEA), poly[2-(dimethylamino)ethyl methacrylate] (p-DMAEMA), poly[2-(diethylamino)ethyl methacrylate] (p-DEAEMA), etc. For example, the polymer may include the following repeating units.

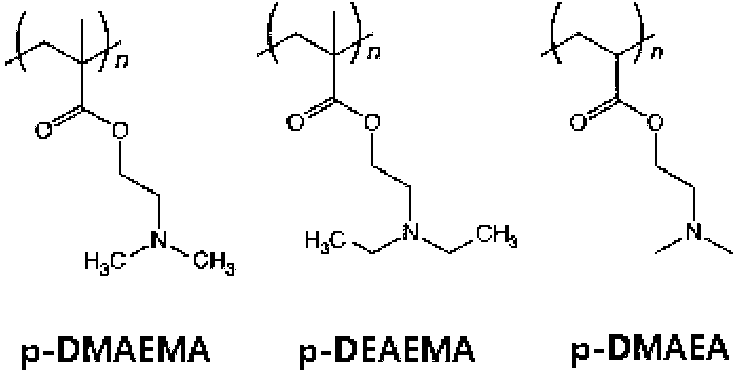

According to an embodiment, the polymer may include p-DMAEA preferably in views of hydrolysis efficiency. The tertiary amine group (dialkylamino group) of p-DMAEA has a relatively small size and has no steric hindrance due to the methyl group bonded to the main chain, thereby easily interacting with the ester carbonyl group.

For example, when the polymer is a copolymer, the polymer may be obtained by copolymerization of a first monomer having a hydrolyzable cationic ester as a side chain and a second monomer different from the first monomer.

For example, the first monomer may include DMAEA, DMAEMA, DEAEMA, or a combination thereof.

The second monomer may include a nucleophilic monomer, a cross-linkable monomer, or a combination thereof. The nucleophilic monomer may include a vinyl group, an acrylic group, or a methacrylic group and a tertiary amine group. The cross-linkable monomer may include at least two vinyl groups, acrylic groups or methacryl groups, or may include a vinyl group, acrylic group or methacryl group and an alkyl halide group.

For example, the second monomer may include 4-vinylpyridine (4VP), vinylimidazole (VIDZ), dimethylaminomethylstyrene (DMAMS), vinylbenzylchloride (VBC), chloroethylacrylate (CEA), divinylbenzene (DVB), glycidylmethacrylate (GMA), di(ethylene glycol)divinylether (DEGDVE), ethyleneglycoldimethacrylate (EGDMA), ethyleneglycoldiacrylate (EGDA), 1,4-butanedioldiacrylate (BDDA), 2,4,6,8-Tetramethyl-2,4,6,8-tetravinylcyclotetrasiloxane (V4D4), 1,3,5-trimethyl-1,3,5-trivinyl-cyclotrisiloxane (V3D3), cyclohexyl methacrylate (CHMA), or a combination thereof. For example, when the second monomer includes a cross-linkable monomer such as vinylbenzyl chloride, chloroethyl acrylate, divinylbenzene, glycidyl methacrylate, di(ethylene glycol) divinyl ether, ethylene glycol dimethacrylate, ethylene glycol diacrylate, 1,4-butadienediol diacrylate, 2,4,6,8-tetramethyl-2,4,6,8-tetravinylcyclotetrasiloxane, 1,3,5-trimethyl-1,3,5-trivinylcyclotrisiloxane, etc., the copolymer may have a cross-linked structure.

For example, the molar ratio of the first repeating unit derived from the first monomer and the second repeating unit derived from the second monomer in the copolymer may be 1:0.1 to 1:0.3. When the ratio of the second monomer is excessively high, the hydrolysis efficiency may decrease, thereby lowering the nucleic acid release efficiency.

For example, the thickness of the polymer thin film may be 1 nm to 1,000 nm, or 10 nm to 500 nm, but is not limited thereto, and may be adjusted according to purposes, types of samples, etc.

For example, the polymer thin film may be formed on a substrate by a chemical vapor deposition method. For example, a vaporized monomer may be activated in a reactor to progress a polymerization reaction, and thus the polymer thin film may be coated on a substrate. For example, the substrate may be the body 110. However, embodiments of the present invention are not limited thereto, and the polymer thin film may be coated on an individual substrate and then placed in the body 110 together with the substrate.

According to an embodiment, the polymer thin film may be formed by initiated chemical vapor deposition (iCVD) using an initiator. According to iCVD, a vaporized initiator and a monomer are adsorbed on a surface of a substrate. The initiator may be activated by heating with a filament or by ultraviolet light. A chain polymerization reaction is initiated by free radicals generated by the activated initiator. As a result, a polymer thin film may be formed on the substrate. The above method does not use a solvent, etc., and synthesis and coating are performed in a same process. Therefore, uniformity of a polymer may be increased, and a thin film with a desired thickness may be formed by controlling process variables.

For example, a peroxide such as tert-butyl peroxide (TBPO) may be used as the initiator. However, the embodiments of the present invention are not limited thereto, and a benzophenone-based initiator that may be activated by UV may also be used.

The polymer thin film according to the embodiments of the present invention includes a cationic polymer and has high solubility in water. Therefore, the polymer may capture nucleic acids in a dissolved state during a nucleic acid extraction process. Therefore, the capture area may be greatly increased, thereby increasing the capture efficiency and shortening the capture time.

In addition, the polymer may be hydrolyzed by self-catalysis. Therefore, larger nucleic acids may be released at a lower temperature than the conventional nucleic acid release method using heating.

For example, the nucleic acid extraction vessel having the polymer thin film may be used as a vessel for a nucleic acid extraction step and an amplification step of PCR (polymerase chain reaction).

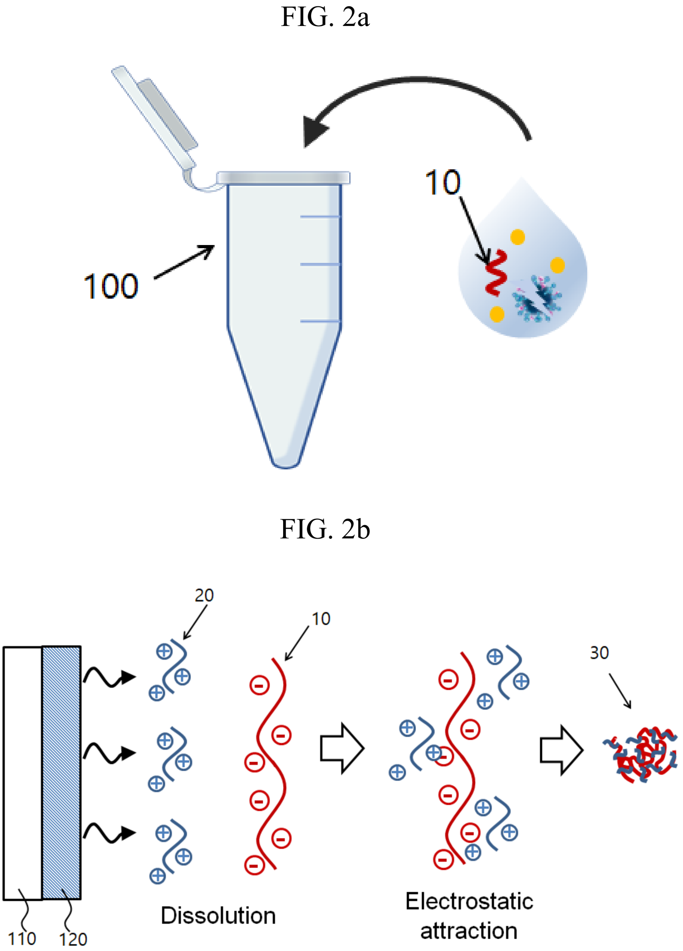

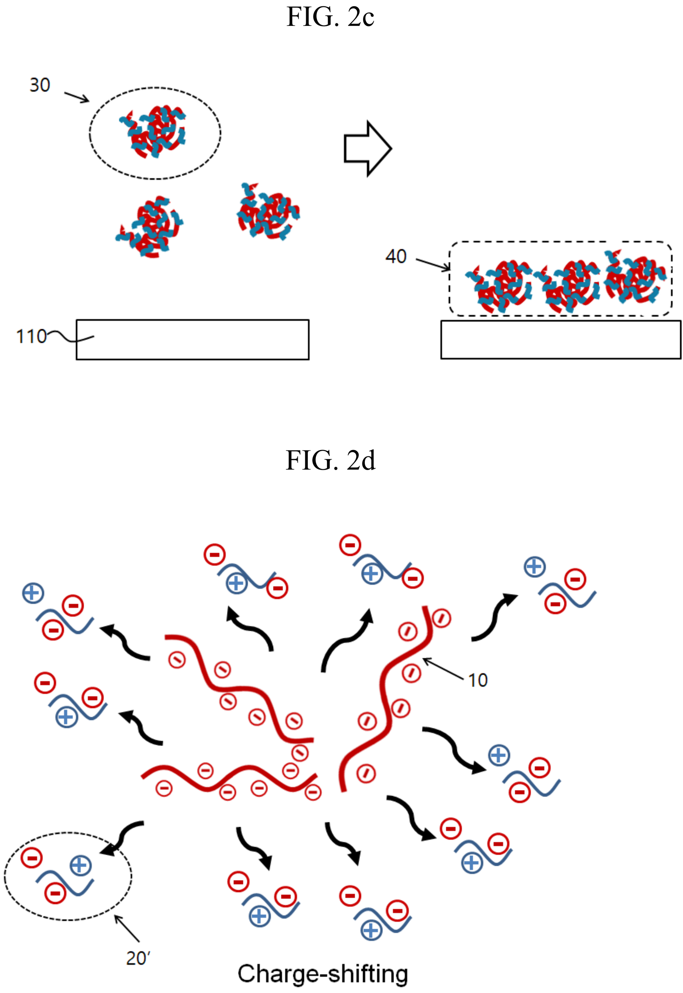

The nucleic acid capture-release mechanism of the polymer will be described in detail below. FIGS. 2a, 2b, 2c, and 2d are schematic diagrams for explaining a nucleic acid extraction method using a nucleic acid extraction vessel according to an embodiment of the present invention.

Referring to FIGS. 2a and 2b, a sample containing a nucleic acid 10 is provided in a nucleic acid extraction vessel 100. The nucleic acid extraction vessel includes a polymer thin film 120 disposed therein. The polymer thin film 120 includes a cationic polymer and has a positive surface potential. The polymer may be easily dissolved by water or the like. The polymer 20 dissolved from the polymer thin film 120 has a positive charge, and the nucleic acid 10 has a negative charge. Therefore, the dissolved polymer 20 may capture the nucleic acid 10 by electrostatic attraction. The dissolved polymer 20 may bind to the nucleic acid 10 by electrostatic attraction to form a polymer-nucleic acid complex (30, polyplex).

According to embodiments of the present invention, the region where electrostatic bonding between the polymer and the nucleic acid occurs is not limited to the two-dimensional surface of the polymer thin film 120, but is extended to the three-dimensional interface of the dissolved polymers 20 and the sample. Therefore, the sampling efficiency and capture speed of the nucleic acid may be significantly increased.

For example, the nucleic acid 10 may include ribonucleic acid (RNA), deoxyribonucleic acid (DNA), methylphosphonate nucleic acid, S-oligo (phosphorothioate oligonucleotide), c-DNA, miRNA, aptamer, etc.

The sample including the nucleic acid 10 may be derived from a biological organism. For example, the sample may include a biological fluid. The biological fluid may include urine, blood, plasma, serum, saliva, semen, feces, sputum, cerebrospinal fluid, tears, mucus, amniotic fluid, etc. In addition, the sample may include biological tissues of humans, animals, plants, bacteria, fungi, and viruses, cells thereof, or intracellular materials thereof. In addition, the sample may include environmental samples such as drinking water, food, etc.

For example, the polymer may be dissolved by the sample. However, embodiments of the present invention are not limited thereto. For example, the polymer may be dissolved by a buffer for dissolving nucleic acids or a sample mixed with the buffer.

The polymer thin film 120 may be completely or partially dissolved. For example, a portion of the polymer thin film may remain on the body 110 of the extraction vessel.

The nucleic acid capture step described above may be performed at room temperature. For example, the above steps may be performed at a temperature of 10 °C or higher and less than 30°C, and preferably at a temperature of 20 °C or higher and less than 30°C. When the temperature of the nucleic acid capture step is excessively high, hydrolysis of the polymer may occur, which may decrease the nucleic acid capture efficiency.

Referring to FIG. 2c, a polymer-nucleic acid complex 30 is precipitated. Centrifugation may be performed to precipitate the polymer-nucleic acid complex 30. For example, as adjacent polymer-nucleic acid complexes 30 coagulate, a precipitate 40 by the coagulated polymer-nucleic acid complexes may be formed.

For example, after precipitation of the polymer-nucleic acid complex 30, a supernatant may be removed to remove foreign substances such as floating substances. After the supernatant is removed, the precipitate 40 may be washed again using a washing buffer, purified water, etc. to further remove foreign substances. Preferably, the precipitation and washing of the polymer-nucleic acid complex 30 may be performed in-situ within the nucleic acid extraction vessel.

Referring to FIG. 2d, the nucleic acid 10 is released from the precipitated polymer-nucleic acid complex. According to embodiments of the present invention, hydrolysis may be used to release the nucleic acid 10.

The polymer may be hydrolyzed by self-catalysis. For example, the repeating unit of the polymer may have a hydrolyzable cationic ester group as a side chain. Self-catalyzed hydrolysis may proceed by intramolecular interaction between the cationic group (e.g., dialkylamino group) and the ester carbonyl group. For example, when the polymer is p-DMAEA, the polymer may be hydrolyzed as follows to be converted into a copolymer of DMAEA and AA (acrylic acid), and dimethylaminoethanol may be generated as reaction byproduct.

### <hydrolysis of p-DMAEA>

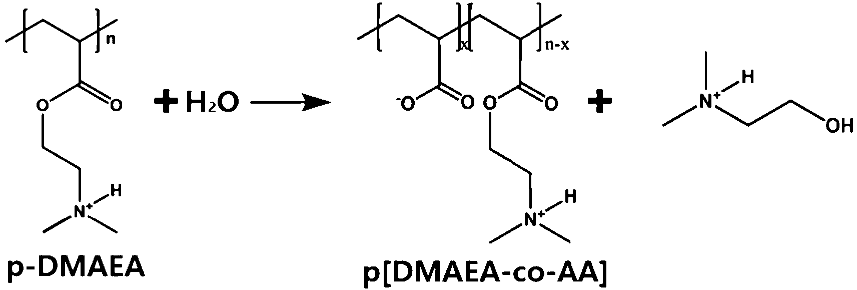

(n and x may be natural numbers, and x is smaller than n.)

The hydrolyzed polymer 20' has a negative charge due to -COO- of an acrylic acid repeating unit. Therefore, when hydrolysis proceeds, the nucleic acid 10 may be released by electrostatic repulsion between the nucleic acid 10 having a negative charge and the polymer 20' having a negative charge.

The hydrolysis may be promoted by heat. However, the hydrolysis may be performed at a temperature much lower than heat treatment required for a conventional nucleic acid release process, and may release a larger amount of nucleic acid at a faster rate. Therefore, the speed, efficiency, and accuracy of the nucleic acid extraction step and the detection method including it may be improved.

For example, the nucleic acid release step by hydrolysis may be performed at 30 °C to 60 °C or 40 °C to 60°C. When the temperature of the hydrolysis is excessively increased, it may affect the subsequent amplification step, etc., and when the temperature is excessively low, the hydrolysis efficiency and hydrolysis speed may be significantly reduced.

According to an embodiment, the nucleic acid release step may be in-situ followed by the amplification step (or reverse transcription step) for nucleic acid detection. For example, the nucleic acid release step may be performed after a PCR mixture is added into the nucleic acid extraction vessel. The polymer does not substantially affect an amplification process or a reverse transcription process. Therefore, it is possible to perform the nucleic acid release step and the amplification step sequentially or simultaneously without a process for separating the polymer. However, the embodiments of the present invention are not limited thereto, and the nucleic acid release step may be performed using a separate buffer, and the amplification step may be performed after the polymer from which the nucleic acid has been released is removed.

For example, the PCR mixture may include a primer, a probe, a dNTP (deoxynucleoside triphospate), which is a mixture of dATP, dTTP, dGTP, and dCTP, a reverse transcriptase, etc.

For example, the nucleic acid release step may be performed in an environment of pH 6 to 9 or pH 7.5 to 8.5, and may be performed for 1 minute to several hours. For example, the hydrolysis efficiency (%) of the polymer may be 30% to 100% when reacted at 50 °C for 1 hour, preferably, may be 30% or more when reacted at 50 °C for 30 minutes, and more preferably, may be 30% or more when reacted at 50 °C for 5 minutes.

After the nucleic acid release step, a polymerase chain reaction may be performed in-situ. For example, the PCR for the amplification step may include at least one of assembly-PCR, asymmetric PCR, digital PCR, endpoint PCR, inverse PCR, methylation-specific PCR, qualitative PCR, quantitative PCR, real-time PCR, and quantitative reverse transcription (qRT)-PCR, but the embodiments of the present invention are not limited thereto, and may be combined with other technologies capable of amplifying nucleic acids in addition to PCR.

Thereafter, an amplified product is analyzed to detect a target nucleic acid. For example, fluorescence derived from the amplified product may be measured to detect the target nucleic acid.

According to embodiments of the present invention, since nucleic acids are captured using a dissolved cationic polymer, an area for polymer-nucleic acid interaction may be expanded. Therefore, the nucleic acid capture efficiency may be improved, and the time required for capturing nucleic acid may be significantly shortened.

In addition, the nucleic acid may be released by electrostatic repulsion between the polymer and the nucleic acid by self-catalytic hydrolysis of the polymer. Therefore, more nucleic acids may be released at a lower temperature than the conventional nucleic acid release method without changing conditions that may affect the amplification step.

In addition, the polymer has stability in the amplification step following the nucleic acid extraction step. Therefore, a single-pot detection system that performs the nucleic acid extraction step and the amplification step continuously may be implemented.

### Nucleic acid detection method

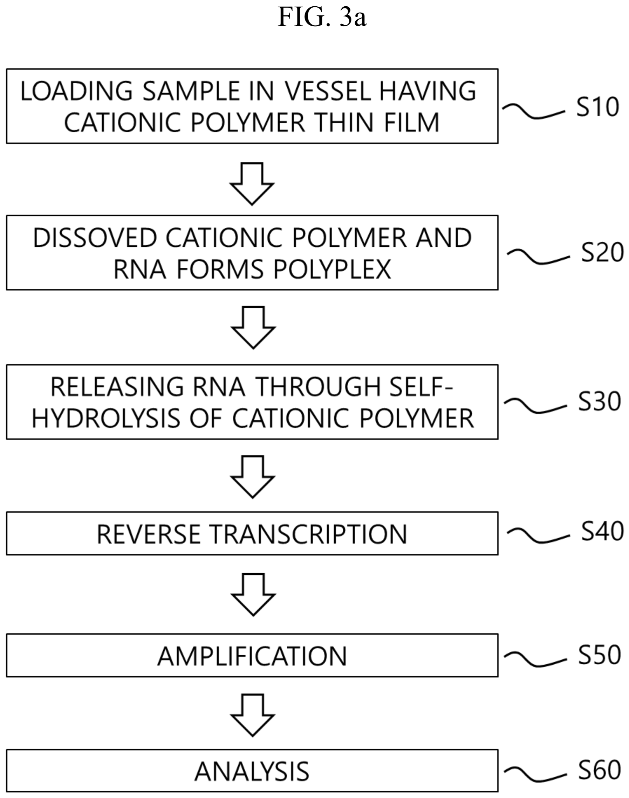

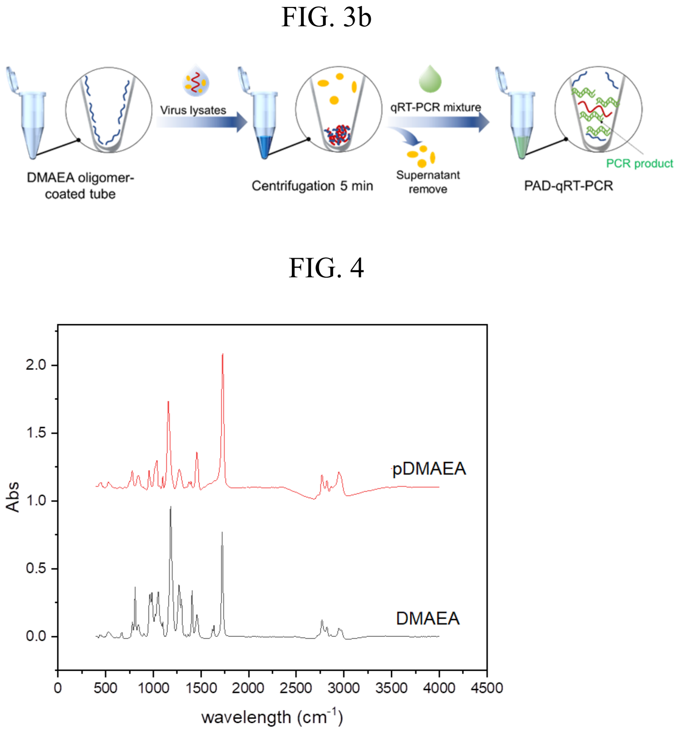

FIG. 3a is a flowchart for explaining a nucleic acid detection method according to an embodiment of the present invention. FIG. 3b is a schematic diagram for explaining a nucleic acid detection method according to an embodiment of the present invention.

According to the nucleic acid detection method according to an embodiment of the present invention, firstly, a sample containing a nucleic acid is loaded into an extraction vessel having a cationic polymer thin film (S10).

The cationic polymer thin film and the extraction vessel may be the same as those described in the above. In an embodiment, the sample may include virus lysates, and the nucleic acid may include RNA of a respiratory virus.

For example, the respiratory virus may include Corona Virus 229E, Corona Virus OC43, Corona Virus NL63, Influenza A Virus, Influenza B Virus, Parainfluenza Virus 1, Parainfluenza Virus 2, Parainfluenza Virus 3, Respiratory Syncytial Virus A, Respiratory Syncytial Virus B, Adeno Virus, Rhino Virus A, B, C, Metapneumovirus, Boca Virus, etc.

Thereafter, the nucleic acid is captured using the cationic polymer film (S20). Specifically, a cationic polymer dissolved from the cationic polymer film may bind to the nucleic acid by electrostatic attraction to form a polyplex. The polyplex may be coagulated and precipitated.

The nucleic acid capture and precipitation may be performed through centrifugation. For example, the centrifugation may be performed for about 1 minute to 30 minutes.

Thereafter, the captured RNA is released through self-hydrolysis of the cationic polymer (S30). In addition, cDNA (complementary DNA) is synthesized from the RNA through a reverse transcription reaction (S40). The RNA release step and the reverse transcription step may be performed simultaneously. The self-hydrolysis of the cationic polymer may occur at a lower temperature than a normal hydrolysis, and the cationic polymer does not affect reverse transcription. Therefore, the RNA may be released at a temperature for performing reverse transcription, and reverse transcription reaction may proceed simultaneously. For example, the RNA release and the reverse transcription may be performed at 30 °C to 60 °C or 40 °C to 60°C.

Before the RNA release step and the reverse transcription step, supernatant of the vessel may be removed, and then the PCR mixture may be added into the vessel. For example, the PCR mixture may include a reverse transcriptase, a dNTP (substrate), and a primer. In addition, the PCR mixture may further include a DNA polymerase and a probe.

The primer may refer to a short nucleic acid sequence having a short free 3' hydroxyl group, which may form a base pair with a complementary template and serve as a starting point for copying a template strand. The primer may initiate DNA synthesis in the presence of a reagent (DNA polymerase or reverse transcriptase) and dNTP for polymerization reaction in an appropriate buffer solution and at an appropriate temperature.

If necessary, the primer may include a label that may be detected directly or indirectly by spectroscopic, photochemical, biochemical, immunochemical or chemical means. Examples of the label may include enzymes, radioisotopes, fluorescent molecules, etc.

The probe may refer to a nucleic acid fragment of RNA or DNA, ranging from several bases to several hundred bases, which may specifically bind to a complementary base sequence, and is labeled so that the presence or absence of a specific base sequence may be confirmed. The probe may include an oligonucleotide probe, a single stranded DNA probe, a double stranded DNA probe, an RNA probe, etc.

Amplification may be performed after the reverse transcription step (S50). The cDNA may be amplified in the amplification step. The amplification step may be performed in the same vessel following the RNA release and reverse transcription steps. The amplification step may be performed at a higher temperature than the reverse transcription step. For example, the amplification step may be performed at 70 °C to 90°C.

Thereafter, an amplification product is analyzed (S60). Accordingly, information on the presence and amount of the detection target may be obtained.

The nucleic acid detection method of the present invention may be performed in the same vessel for the nucleic acid extraction step and the amplification step (including reverse transcription), and may be performed under substantially the same process and under the same conditions as conventional qRT-PCR. Therefore, commercially available amplification kits, etc. may be used, and conventional detection methods may be easily replaced.

Hereinafter, the synthesis and effects of polymer thin films according to exemplary embodiments will be described in detail through specific experimental examples. However, the above experimental examples are provided merely as examples, and the scope of the present invention is not limited to the contents provided in the above experimental examples.

### Synthesis Example 1

The commercial PCR tube (Bio-Rad Laboratories, Inc., USA) was placed on the substrate of the iCVD chamber, and the temperature of the substrate was maintained at 30 °C . Thereafter, DMAEA and an initiator TBPO were vaporized at a ratio (feed ratio) of 80:30 mtorr while maintaining the temperature at 35 °C and 25 °C , respectively, and transferred to the chamber. The pressure inside the chamber was maintained as a vacuum state of 150 mTorr, and at the same time, the filament was heated to 140 °C to radically polymerize the monomers adsorbed on the substrate, thereby producing a DMAEA homopolymer (film thickness: about 350 nm).

FIG. 4 is a graph showing the results of FTIR (Fourier transform infrared) analysis of the DMAEA polymer (pDMAEA) and monomer (DMAEA) of Synthesis Example 1.

Referring to FIG. 4, the 1626 cm-1 peak indicating a vinyl group was smaller in the polymer (pDMAEA) compared to the monomer (DMAEA). Therefore, it can be noted that the polymer was well synthesized (the vinyl group of the monomer disappears as polymerization progresses).

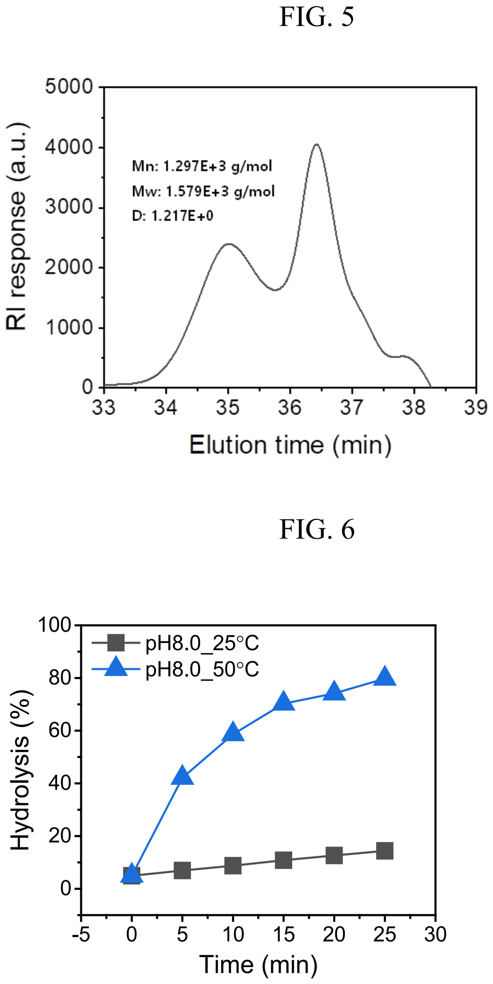

FIG. 5 is a graph showing the results of GPC (Gell permeation chromatography) analysis of the DMAEA polymer of Synthesis Example 1. Referring to FIG. 5, the number average molecular weight (Mn) of the DMAEA polymer of Synthesis Example 1 was approximately 1,300 g/mol.

### Hydrolysis experiment

The DMAEA polymer of Synthesis Example 1 was hydrolyzed at pH 8.0, 25°C, and 50°C, and the hydrolysis efficiency was calculated over time (at 5-minutes intervals). Specifically, the hydrolysis efficiency was calculated by comparing the sum of CH₂O-methylene signals corresponding to the non-hydrolyzed side chain group and the sum of alcohol signals corresponding to reaction by-product using NMR (Nuclear magnetic resonance) spectroscopy.

FIG. 6 is a graph showing the hydrolysis efficiency (%) over time (at 5-minutes intervals) when the DMAEA polymer of Synthesis Example 1 was hydrolyzed at pH 8.0, 25°C, and 50°C. Referring to FIG. 6, it can be noted that the hydrolysis of the DMAEA polymer of Synthesis Example 1 can be actively performed at a temperature of 30 °C or higher, and it was confirmed that the hydrolysis efficiency was 80% or higher after 25 minutes at 50°C. In particular, it can be noted that more than 45% of hydrolysis was achieved within 5 minutes at 50°C. Through this, it can be noted that release of RNA may proceed rapidly in the reverse transcription step.

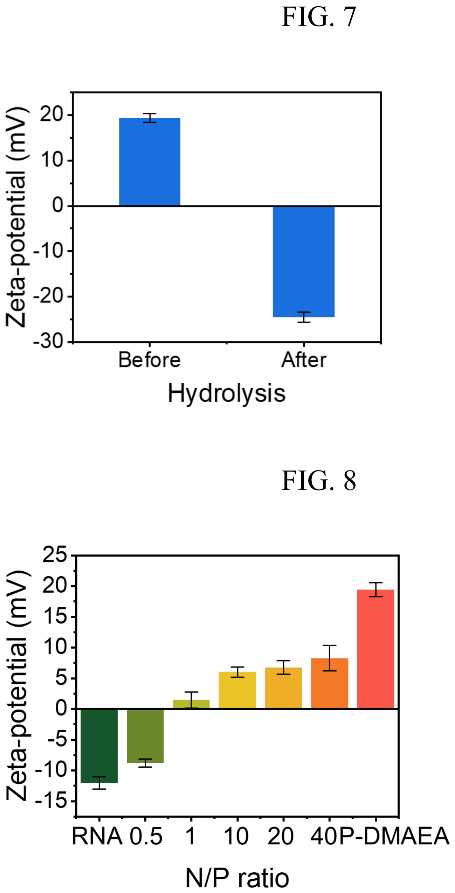

FIG. 7 is a graph showing the zeta-potential before and after hydrolysis of the DMAEA polymer of Synthesis Example 1. Referring to FIG. 7, the zeta-potential of the DMAEA polymer of Synthesis Example 1 was 20 mV before hydrolysis, but changed to -25 mV after hydrolysis, which may be due to increase of AA groups having a negative charge due to hydrolysis.

FIG. 8 is a graph showing the zeta-potential according to the ratio of the DMAEA polymer and RNA (N(nitrogen)/P(phosphate) ratio) of Synthesis Example 1. Referring to FIG. 8, the zeta-potential value increases as the ratio of the DMAEA polymer increases, and when the N/P ratio is 1, the zeta-potential value changes rapidly to have a positive value (+).

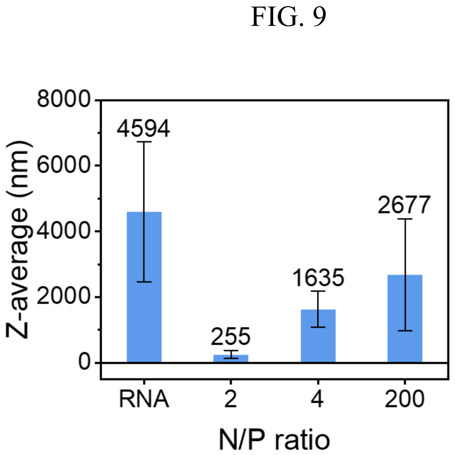

FIG. 9 is a graph showing the average hydrodynamic diameter (Z-average) according to the ratio of the DMAEA polymer and RNA (N(nitrogen)/P(phosphate) ratio) of Synthesis Example 1. Referring to FIG. 9, when the N/P ratio is 2, the Z-average value decreases sharply to 255 nm, which confirms that a polyplex was formed.

### Nucleic acid capture experiment

As a model RNA, the influenza virus provided by the Korea Research Institute of Bioscience and Biotechnology (KRIBB) was selected. HINI virus RNA was prepared using a G-spin genomic RNA extraction kit (iNtRON Biotechnology, Korea) according to the manufacturer's protocol. The concentration of the extracted RNA was determined with a NanoDrop^{™} 1000 spectrometer (Thermo Fisher Scientific, USA). In order to measure the amount of captured RNA, 3 µL of RNA solution (concentration: 50, 20, 10, 1 ng/uL) was added to the tube coated with the polymer obtained in Synthesis Example 1. After centrifugation at room temperature for 5 minutes, uncaptured RNA was recovered. The concentration of uncaptured RNA was measured through qRT-PCR.

The PCR mixture used included the uncaptured RNA solution (supernatant, 1 µL), primer set (each 0.6 µM), QIAGEN One-Step RT-PCR Buffer (Tris-Cl, KCl, (NH₄)₂SO₄, 12.5 mM MgCl₂, DTT, pH 8.7), QIAGEN One-Step RT-PCR Enzyme Mix (1 µL), and dNTP Mix (400 µM). PCR amplification was performed using a CFX Connect^{™} Real-Time System (Bio-Rad, USA) with the following conditions: reverse transcription at 50 °C for 30 min, initial denaturation at 95 °C for 5 min, thermal cycles of 95 °C for 30 seconds, 60 °C for 60 seconds, and 72 °C for 30 seconds for 40 cycles, and a final extension at 72 °C for 5 min. The amount of the captured RNA was obtained by subtracting the amount of the uncaptured RNA from the amount of the input RNA.

The sequences of the primers used in the following experiments are shown in Table 1 below.

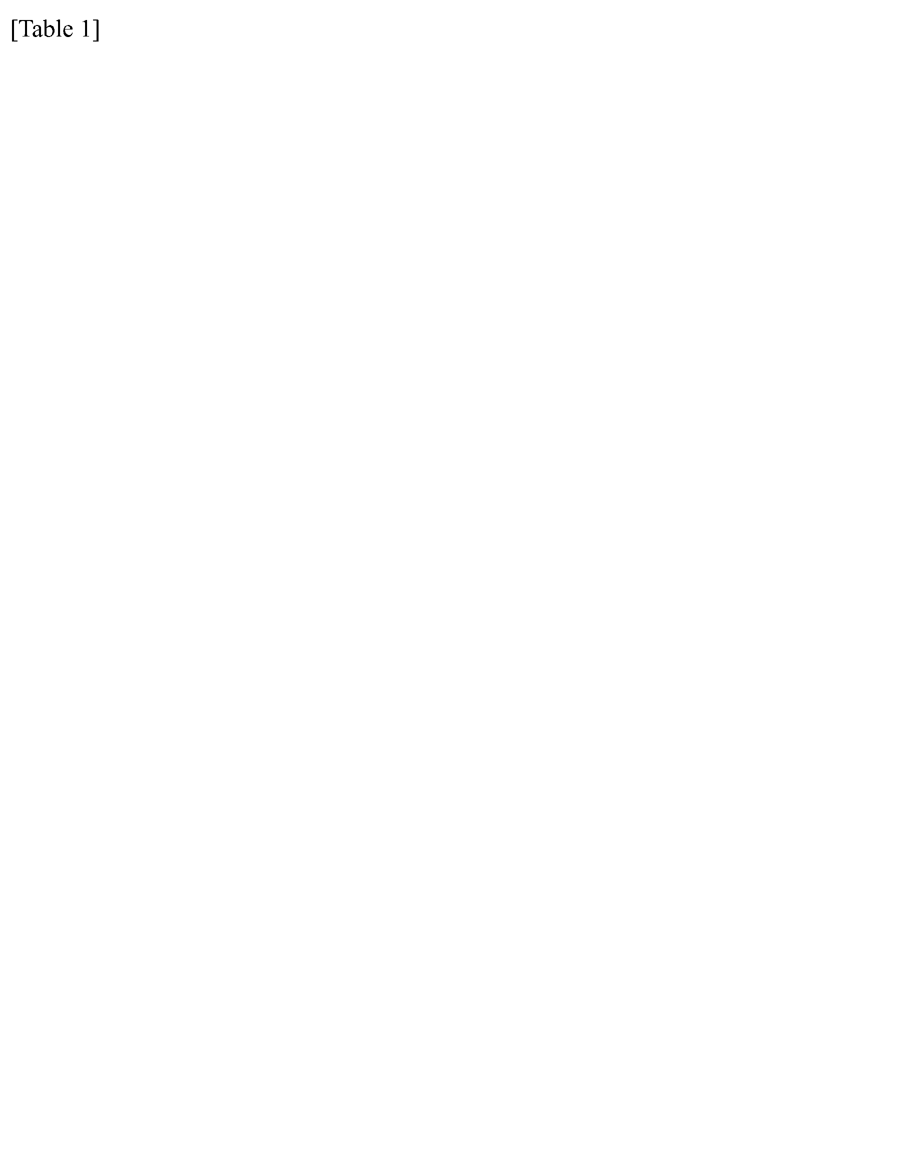

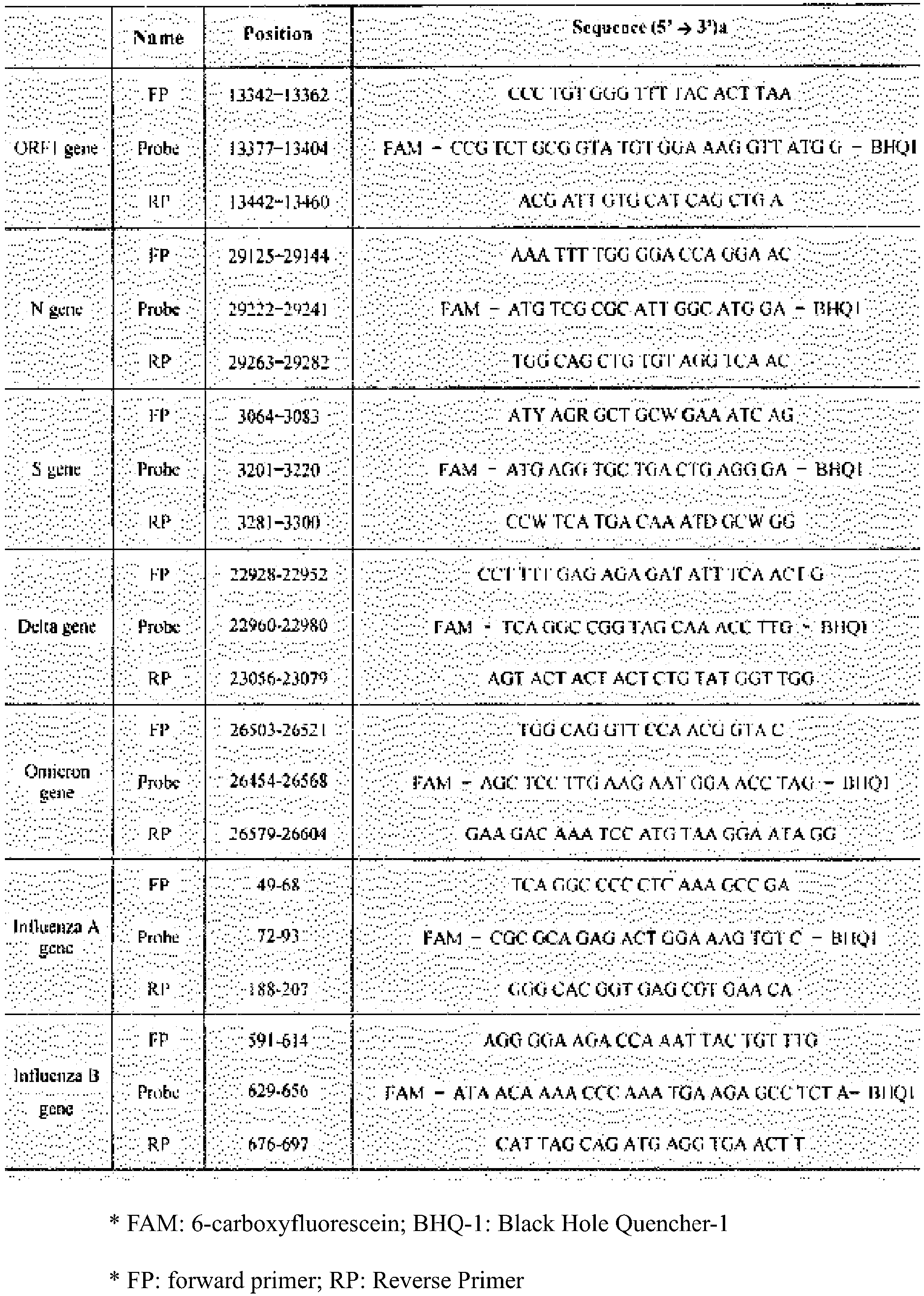

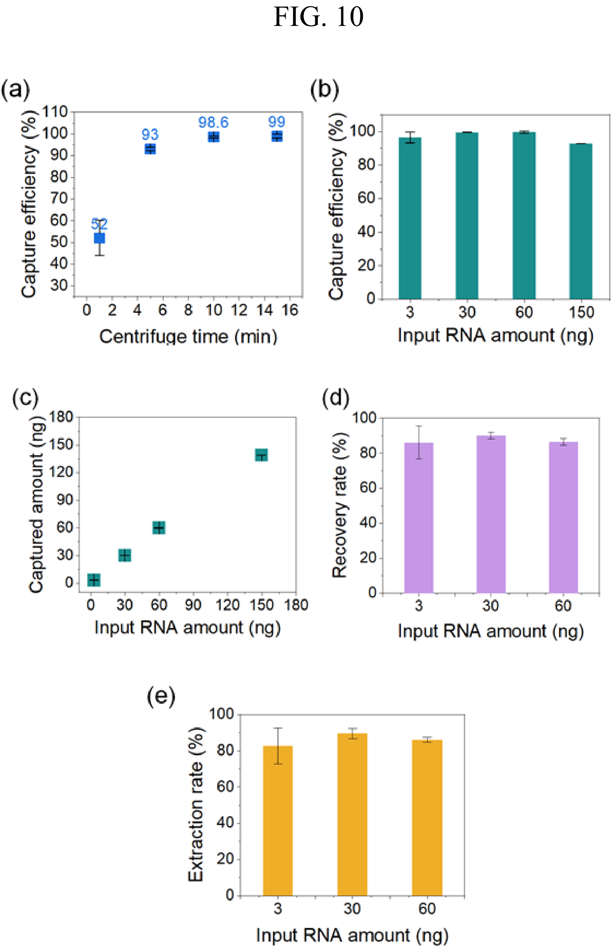

FIG. 10 is a graph showing the capture efficiency, capture amount, recovery rate, and extraction rate in the nucleic acid capture experiment using the tube coated with the DMAEA polymer of Synthesis Example 1. (a) is a graph showing the capture efficiency {(captured RNA/input RNA) × 100} according to the centrifugation time, (b) is a graph showing the capture efficiency (centrifugation: 5 minutes) according to the amount of input RNA, and (c) is a graph showing the input amount according to the amount of input RNA.

Referring to (a), 99% of RNA was finally captured, and 93% of RNA was captured by 5 minutes of centrifugation. This rapid RNA capture characteristic may be due to rapid dissolution of the polymer (oligomer) on the surface of the tube.

Furthermore, referring to (b), high capture efficiency was maintained for various input RNA amounts (3, 30, 60, and 150 ng), and referring to (c), the capture amount increased linearly with the input RNA amount, which confirms the effective RNA capture ability of the DMAEA polymer-coated tube.

In order to utilize the DMAEA polymer-coated tube for single-pot genetic analysis, it is necessary to rapidly release RNA from the polyplex in the initial stage of qRT-PCR. In order to examine the amount of RNA released from the polyplex, a sample was loaded into a DMAEA polymer-coated tube, centrifugation was performed, the supernatant was removed, and qRT-PCR (same conditions as the above) was performed in the tube. In FIG. 10, (d) is a graph showing the recovery rate {(quantified RNA/captured RNA) × 100} according to the amount of input RNA, and (e) is a graph showing the extraction rate {(quantified RNA/input RNA) × 100} according to the amount of input RNA.

Referring to (d) and (e), the recovery rate of the DMAEA polymer-coated was about 85%, and the extraction rate was 80% or more. Considering that the recovery rate of the commercially available spin column extraction kit is about 20%, it can be noted that RNA can be detected with high efficiency and accuracy using the tube of the present invention, and that the tube of the present invention may be applied to general qRT-PCR without separate chemical treatment or condition change.

### Virus detection experiment: Influenza

In order to test a detection method (polyplex-assisted direct (PAD) qRT-PCR) according to an embodiment of the present invention, influenza A virus (H1N1) was detected in the same manner as the nucleic acid capture experiment.

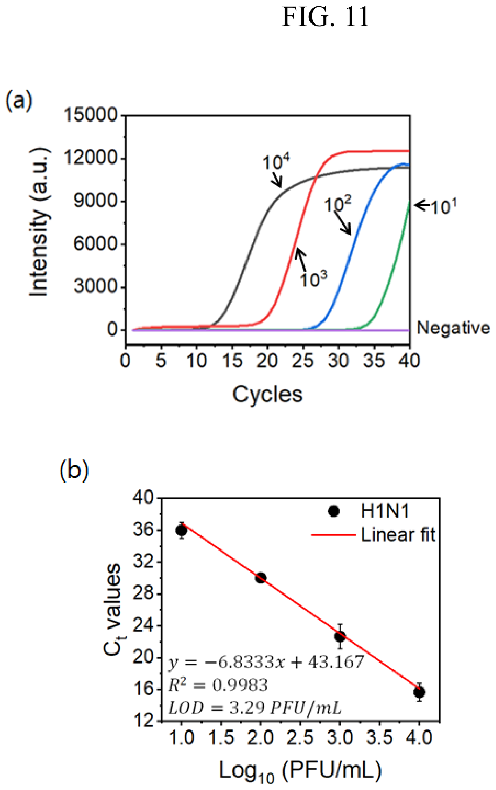

FIG. 11 shows the detection results of H1N1 using the detection method (PAD-qRT-PCR) of the present invention. In FIG. 11, (a) shows the amplification curves for various concentrations of H1N1, and (b) shows the corresponding Ct values plotted as a function of H1N1, which shows the dynamic detection range of plaque forming units (PFU/ml) from 10 to 10⁴.

Referring to FIG. 11, the correlation coefficient (R²) of the linear regression line is 0.9983, which confirms that quantification of H1N1 is possible using the detection method of the present invention. The detection sensitivity of H1N1 in the detection method of the present invention was calculated to be 3.29 PFU/mL, which is comparable to or much lower than the minimal infective dose (MID, 10 ^{0.5-1.5} PFU) of H1N1, supporting the possibility of detecting H1N1 in the range of clinical demand.

### Virus detection experiment: Influenza - clinical diagnosis

Influenza virus in clinical samples was detected for further experiments with single-pot PAD-qRT-PCR. Specifically, samples diagnosed at the Severance Hospital Yonsei University Health Service Center (collected and diagnosed before the COVID-19 pandemic, then stored at -70 °C , H1N1: 11, IBV: 9, negative: 15) were obtained, and 10 *µ*ℓ of each sample was combined with 90 *µ*ℓ of lysis buffer (containing proteinase K (0.8 U/reaction) and RNase inhibitor (10 U/reaction)), pulse-vortex mixed for 15 seconds, and incubated at room temperature for 10 minutes. 3 *µ*ℓ of the above lysate solution was placed in the tube of Synthesis Example 1, centrifuged for 5 minutes, and the supernatant was removed. Thereafter, a PCR mixture was added, and PCR was performed.

The PCR mixture used was a 25 µL solution containing a primer set (each 0.6 µM), QIAGEN One-Step RT-PCR Buffer, QIAGEN One-Step RT-PCR Enzyme Mix (1 µL), and dNTP mixture (400 µM). PCR amplification was performed with reverse transcription at 50 °C for 20 minutes, initial denaturation at 95 °C for 15 minutes, and then 40 cycles of 95 °C for 10 seconds, 60 °C for 20 seconds, and 72 °C for 30 seconds.

Table 2 below shows the results diagnosed by PAD-qRT-PCR.

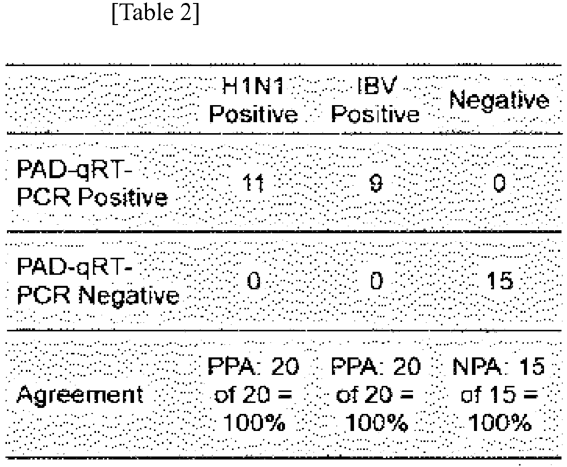

Referring to Table 2, it can be confirmed that the results diagnosed by PAD-qRT-PCR are consistent with the results diagnosed in the clinical hospital.

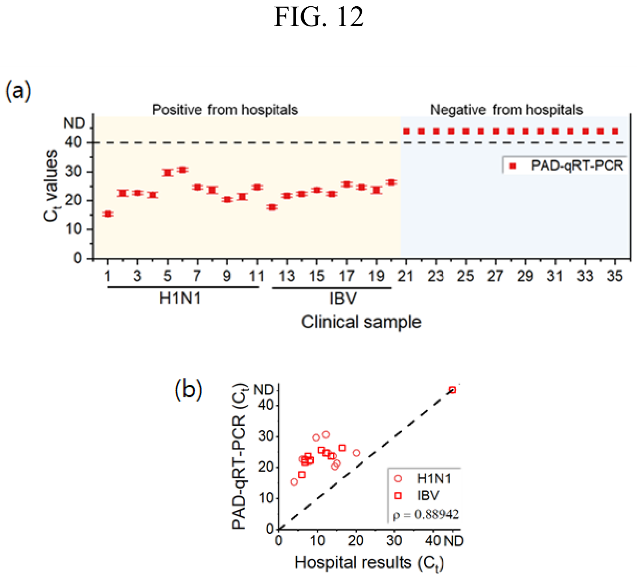

FIG. 12 shows the results of analyzing clinical samples (influenza virus) using the detection method (PAD-qRT-PCR) of the present invention. In FIG. 12, (a) is a graph showing the Ct values for each sample, and (b) is a scatter plot of the Ct values obtained using the detection method (PAD-qRT-PCR) of the present invention with respect to the C values provided by the hospital.

Referring to FIG. 12, the Ct values obtained using the detection method (PAD-qRT-PCR) of the present invention were higher than the values provided by the hospital, but this was due to the difference in measurement time (3-4 years), and it can be confirmed that accurate diagnosis was possible even with long-term stored samples.

### Virus detection experiment: SARS-CoV-2

In order to test the detection method (PAD-qRT-PCR) according to the embodiment of the present invention, the corona virus (SARS-CoV-2) was detected using the spike (S) gene primer (Table 1).

Specifically, 90 *µ*ℓ of the virus sample was treated with 10 *µ*ℓ of TCEP (tris(2-carboxyethyl) phosphine)/EDTA (ethylenediaminetetraacetic acid) (final concentrations of 100 and 1 mM, respectively), and then heated at 50 °C for 5 minutes and 64 °C for 5 minutes to prepare a SARS-CoV-2 virus lysate. 100 *µ*ℓ of the virus lysate was treated with proteinase K (0.8 U/reaction) and RNase inhibitor (10 U/reaction), pulse-vortex mixed for 15 seconds, and incubated at room temperature for 10 minutes. 3 *µ*ℓ of the lysate solution was placed in the tube of Synthesis Example 1, centrifuged for 5 minutes, and the supernatant was removed. Thereafter, a PCR mixture was added, and PCR was performed.

The PCR mixture used was a 25 µL solution containing a primer set (each 0.6 µM), QIAGEN One-Step RT-PCR Buffer, QIAGEN One-Step RT-PCR Enzyme Mix (1 µL), and dNTP Mix (400 µM). PCR amplification was performed by heating at 95 °C for 2 minutes, then 40 cycles of 95 °C for 10 seconds, 55 °C for 15 seconds, and 72 °C for 35 seconds.

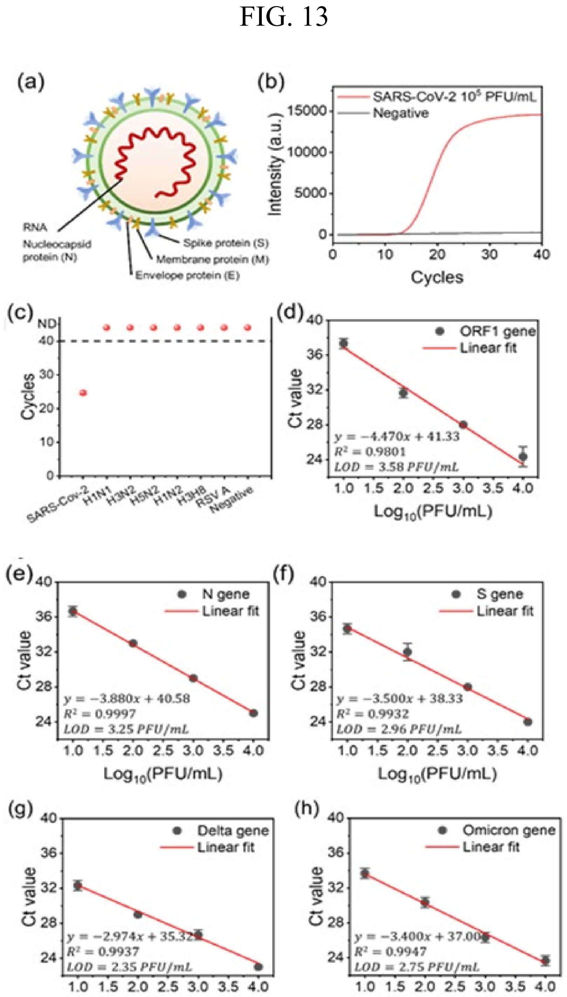

FIG. 13 shows a schematic diagram of SARS-CoV-2 and the results of the detection experiment. Referring to FIG. 13 (b), when SARS-CoV-2 (10⁵ PFU/mL) was present, the fluorescence signal increased, whereas no signal was detected when the virus was not present. In order to test the specificity of the present invention, other respiratory viruses (subtypes H1N1, H3N2, H5N2, H1N2, and H3H8 of influenza A virus, RSV A) were used for testing, and as shown in (c), it was confirmed that Ct values were not obtained for other viruses.

The experiment was conducted by expanding the SARS-CoV-2 target gene to the open reading frame 1 gene (ORF1 gene) and the nucleocapsid gene (N gene). (d), (e), and (f) in FIG. 13 are graphs showing Ct values for each gene. In each graph, the Ct values decreased as the virus concentration increased, and it was confirmed that they had an excellent linear relationship (R² = 0.9801, 0.9997, and 0.9932 for ORF1, N, and S genes) in the range of 10¹ to 10⁴ PFU/ml. In addition, the limits of detection (LOD) were calculated as 3.58 PFU/ml, 3.25 PFU/ml, and 2.96 PFU/ml for ORF1, N, and S genes, respectively.

In addition, in order to test detection of SARS-CoV-2 variants (Delta and Omicron), experiments were conducted using spike gene (S gene) primers for Delta and Omicron variants. (g) and (h) in FIG. 13 are graphs showing Ct values for Delta and Omicron. Referring to (g) and (h) in FIG. 13, R² for Delta = 0.9937, R² for Omicron = 0.9947, and the limits of detection (LOD) were 2.35 PFU/ml (Delta) and 2.75 PFU/ml (Omicron). Through this, it can be confirmed that the detection method of the present invention (PAD-qRT-PCR) can be used to identify variants of SARS-CoV-2.

### Virus detection experiment: SARS-CoV-2 - clinical diagnosis

135 clinical samples provided by Kyung Sang University College of Medicine and Severance Hospital Yonsei University Health Service Center were analyzed using the detection method of the present invention (PAD-qRT-PCR) described in the above. In addition, as a comparative example, the same clinical samples were analyzed using conventional qRT-PCR using an extraction method based on spin column.

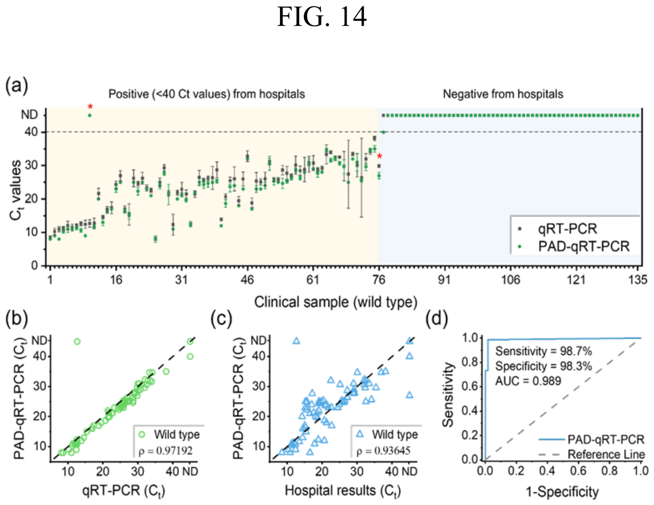

FIG. 14 shows the results of analyzing clinical samples (SARS-CoV-2) using the detection method of the present invention (PAD-qRT-PCR) and the conventional detection method (qRT-PCR). In FIG. 14, (a) is a graph showing the Ct value for each sample, (b) is a scatter plot of the Ct value obtained by the detection method of the present invention (PAD-qRT-PCR) with respect to the Ct value obtained by the conventional detection method (pRT-PCR), (c) is a scatter plot of the Ct value obtained by the detection method of the present invention (PAD-qRT-PCR) targeting the N, S, and ORF1a genes with respect to the average value obtained by targeting the RdRP, E, and N genes in a hospital, and (d) is a ROC (receiver operating characteristic) curve of the patient sample data obtained by the detection method of the present invention (PAD-qRT-PCR).

Referring to FIG. 14, according to the Ct values obtained by the detection method (PAD-qRT-PCR) of the present invention, 74 out of 75 positive predictions were consistent, and 59 out of 60 negative predictions were consistent (samples 10 and 76 showed false negatives and false positives, respectively). In addition, the Ct values obtained by the detection method (PAD-qRT-PCR) of the present invention were generally consistent with the Ct values obtained by the conventional detection method (pRT-PCR) (ρ=0.97192). The detection method (PAD-qRT-PCR) of the present invention provided the Ct value that was 1.35 lower on average than the conventional detection method (pRT-PCR), which is thought to be because the polyplex-based assay has a higher RNA extraction efficiency (about 80%) than the RNA extraction efficiency of the conventional RNA extraction kit (20% to 60%). Assuming that the amplification efficiency of both analysis methods is 100%, a difference of 1.35 in the Ct value means that 2.5 times more RNA was quantified. In addition, the Ct value obtained by the detection method (PAD-qRT-PCR) of the present invention had high correlation with the value obtained in the hospital (ρ=0.93645), and the AUC (area under curve) value according to the ROC analysis result was 0.989, thereby confirming that the accuracy for the clinical condition of the patient is high.

### Virus detection experiment: SARS-CoV-2 variants - clinical diagnosis

Delta variant samples and Omicron variant samples were collected from SARS-CoV-2 patients, and the samples collected before the emergence of the delta variant were adopted as negative controls, and an experiment (by same method as the above) for the detection method (PAD-qRT-PCR) of the present invention was conducted.

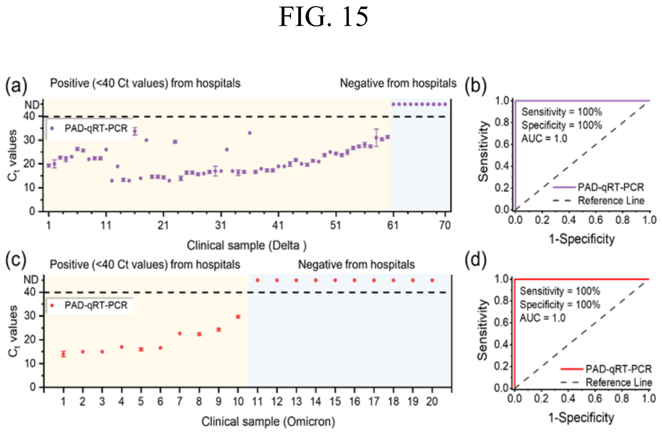

FIG. 15 shows the results of analyzing clinical samples (SARS-CoV-2 variants) using the detection method (PAD-qRT-PCR) of the present invention. In FIG. 15, (a) is a graph showing Ct values for the delta variant samples (including the negative controls), (b) is an ROC curve obtained from delta variant clinical results, (c) is a graph showing Ct values for the omicron variant samples (including the negative controls), and (d) is an ROC curve obtained from omicron variant clinical results.

Referring to FIG. 15, it was confirmed that both positive prediction agreement (60/60) and negative prediction agreement (10/10) for delta variants were 100%, which showed high correlation with the results from the hospital, and the AUC value was 1.0, which showed high accuracy.

As described above, although the present invention has been described with reference to exemplary embodiments, it will be understood by those skilled in the art that the present invention can be variously modified and changed within the scope and spirit of the present invention described in the following patent claims.

### ABILITY OF INDUSTRIAL UTILITY

Embodiments of the present invention can be used for various nucleic acid extraction methods using nucleic acids and target detection, molecular diagnosis, etc. using the same. For example, they can be used for detection and quantification of pathogens such as viruses and bacteria, investigation of the origin of food materials, diagnosis and monitoring of diseases, etc.

### <explanation for reference numerals>

- 10:: nucleic acid
- 20:: dissolved polymer
- 30:: polymer-nucleic acid complex (polyplex)
- 100:: nucleic acid extraction vessel
- 110:: body
- 120:: polymer thin film

## Claims

1. A nucleic acid extraction vessel comprising:
a body for containing a sample; and
a polymer thin film coated on an inner surface of the body,
wherein the polymer thin film includes a cationic polymer having a hydrolyzable side chain, and has a surface potential of 1 mV to 50 mV, and a hydrolysis efficiency of the cationic polymer is 30% or more upon when reacted at 50 °C for 1 hour.

2. The nucleic acid extraction vessel according to claim 1, wherein the hydrolyzable side chain includes a hydrolytic cationic ester.

3. The nucleic acid extraction vessel of claim 2, wherein the cationic polymer includes a repeating unit polymerized from a monomer including at least one of 2-(dimethylamino)ethyl acrylate (DMAEA), 2-(dimethylamino)ethyl methacrylate (DMAEMA), and 2-(diethylamino)ethyl methacrylate (DEAEMA).

4. The nucleic acid extraction vessel of claim 2, wherein the cationic polymer includes a repeating unit polymerized from DMAEA.

5. The nucleic acid extraction vessel of claim 4, wherein the cationic polymer is a linear polymer.

6. The nucleic acid extraction vessel of claim 5, wherein a number average molecular weight of the cationic polymer is 1,000 g/mol to 2,000 g/mol.

7. The nucleic acid extraction vessel of claim 1, wherein a number average molecular weight of the cationic polymer is 1,000 g/mol to 3,000 g/mol.

8. A nucleic acid extraction vessel comprising:
a body having a containing space; and
a polymer thin film coated on an inner surface of the body to contact the containing space,
wherein the polymer thin film includes a cationic polymer having a hydrolyzable side chain, which includes a hydrolytic cationic ester, and having a number average molecular weight of the cationic polymer is 1,000 g/mol to 3,000 g/mol.

9. The nucleic acid extraction vessel of claim 8, wherein the cationic polymer includes a repeating unit polymerized from a monomer including at least one of 2-(dimethylamino)ethyl acrylate (DMAEA), 2-(dimethylamino)ethyl methacrylate (DMAEMA), and 2-(diethylamino)ethyl methacrylate (DEAEMA).

10. The nucleic acid extraction vessel of claim 8, wherein the cationic polymer includes a repeating unit polymerized from DMAEA.

11. The nucleic acid extraction vessel of claim 8, wherein the cationic polymer is a linear polymer and has a number average molecular weight of 1,000 g/mol to 2,000 g/mol.

12. The nucleic acid extraction vessel of claim 8, wherein the body has a tube shape.

13. The nucleic acid extraction vessel f claim 8, wherein a thickness of the polymer thin film is 1 nm to 1,000 nm.

14. A nucleic acid detection method comprising:
inputting a sample containing a nucleic acid into a vessel coated with a thin film including a cationic polymer having a hydrolyzable side chain;
combining the cationic polymer dissolved from the thin film with the nucleic acid to form a polymer-nucleic acid complex;
hydrolyzing the cationic polymer to release the nucleic acid from the polymer-nucleic acid complex; and
amplifying the nucleic acid or another nucleic acid synthesized from the nucleic acid.

15. The nucleic acid detection method of claim 14, wherein hydrolyzing the cationic polymer to release the nucleic acid is performed at 30 °C to 60 °C.

16. The nucleic acid detection method of claim 14, wherein the nucleic acid includes an RNA.

17. The nucleic acid detection method of claim 16, further comprising synthesizing a complementary DNA (cDNA) of the RNA through a reverse transcription reaction, and amplifying the nucleic acid or another nucleic acid synthesized from the nucleic acid amplifies the cDNA.

18. The nucleic acid detection method of claim 17, wherein the nucleic acid is released when the cDNA is synthesized.

19. The nucleic acid detection method of claim 14, wherein the cationic polymer includes a repeating unit polymerized from DMAEA, and a number average molecular weight of the cationic polymer is 1,000 g/mol to 3,000 g/mol.

20. The nucleic acid detection method of claim 14, wherein forming the polymer-nucleic acid complex, releasing the nucleic acid, and amplifying the nucleic acid or another nucleic acid synthesized from the nucleic acid are performed in the same vessel.
